(19) Europäisches Patentamt
European Patent Office
Office européen des brevets

(11) **EP 4 708 301 A1**

(12) **EUROPEAN PATENT APPLICATION**
published in accordance with Art. 153(4) EPC

(43) Date of publication:
**11.03.2026 Bulletin 2026/11**

(21) Application number: 24822238.2

(22) Date of filing: **04.02.2024**

(51) International Patent Classification (IPC):
**G16B 15/30** (2019.01)    **G16B 15/20** (2019.01)
**G16B 40/00** (2019.01)

(52) Cooperative Patent Classification (CPC):
Y02A 90/10

(86) International application number:
**PCT/CN2024/075814**

(87) International publication number:
**WO 2024/255278 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.06.2023 CN 202310714271**

(71) Applicant: **Zhejiang Lab**
**Hangzhou, Zheijiang 311121 (CN)**

(72) Inventors:
• **CHEN, Hongyang**
**Hangzhou, Zhejiang 311121 (CN)**
• **CHEN, Xiangju**
**Hangzhou, Zhejiang 311121 (CN)**
• **AN, Feng**
**Hangzhou, Zhejiang 311121 (CN)**
• **XIAO, Zhu**
**Hangzhou, Zhejiang 311121 (CN)**

(74) Representative: **Dai, Simin**
**Reyda IP**
**A073**
**157, Quai du Président Roosevelt**
**92130 Issy-les-Moulineaux (FR)**

(54) **MODEL TRAINING METHOD AND APPARATUS, AND MOLECULAR PROPERTY INFORMATION PREDICTION METHOD AND APPARATUS**

(57)    The present application provides a model training method and apparatus, and a molecular property information prediction method and apparatus. The method include: obtaining data of a designated proteolysis targeting chimera molecule; according to the data, constructing three-dimensional molecular graph data of the designated proteolysis targeting chimera molecule; inputting the three-dimensional molecular graph data of the designated proteolysis targeting chimera molecule into a prediction model to be trained, so that the prediction model predicts molecular property information of the designated proteolysis targeting chimera molecule; and according to a deviation between predicted molecular property information and actual molecular property information of the designated proteolysis targeting chimera molecule, training the prediction model.

FIG. 1

**Description**

**TECHNICAL FIELD**

The present application relates to the fields of artificial intelligence and bioengineering, and in particular to model training methods and molecular property information prediction methods and apparatuses.

**BACKGROUND**

The ubiquitination enzyme system is a classical pathway for degrading protein in vivo. By using this classical pathway, a proteolysis targeting chimera with bifunctional fragments can be designed, so as to reduce or even eliminate pathogenic protein in vivo. The proteolysis targeting chimera is a targeting protein degradation technology that uses small molecular compounds to regulate protein level. Compared with traditional small molecular drugs, the proteolysis targeting chimera has different modes of action, and can use a unique target to deliver a protein into a proteasome, so as to directly mediate the ubiquitination and degradation process of the protein.

At present, the technology of proteolysis targeting chimera is a popular direction in related pharmaceutical research. Such molecules have been used to develop drugs for treating diseases including cancer, immune system diseases, nervous system diseases, and the like. Especially in terms of anti-cancer, the proteolysis targeting chimera can target protein that induces cancer cell production, thus reducing the side effects caused by chemotherapy drugs.

Molecular property prediction is important for applications such as drug discovery or protein design. By analyzing a molecular structure model to estimate its related physical and chemical properties and calculate accurate molecular properties, the efficiency and accuracy of technical design can be greatly improved. However, at present, there is no effective way to accurately predict molecular properties.

**SUMMARY**

The present application provides a model training method and apparatus, a molecular structure information recommendation method and apparatus, so as to partially solve the above problems existing in the prior art. The present application adopts the following technical schemes.

In the first aspect, the present application provides a model training method, including: obtaining data of a designated proteolysis targeting chimera molecule; according to the data, constructing three-dimensional molecular graph data of the designated proteolysis targeting chimera molecule; inputting the three-dimensional molecular graph data of the designated proteolysis targeting chimera molecule into a prediction model to be trained, so that the prediction model predicts molecular property information of the designated proteolysis targeting chimera molecule; and according to a deviation between predicted molecular property information and actual molecular property information of the designated proteolysis targeting chimera molecule, training the prediction model.

In some embodiments, the molecular property information includes at least one of: lipophilicity of the designated proteolysis targeting chimera molecule, a pH of the designated proteolysis targeting chimera molecule, a molecular weight of the designated proteolysis targeting chimera molecule, a hydrogen bond donor and a hydrogen bond acceptor of the designated proteolysis targeting chimera molecule, a solubility of the designated proteolysis targeting chimera molecule, or a permeability of the designated proteolysis targeting chimera molecule.

In some embodiments, constructing the three-dimensional molecular graph data of the designated proteolysis targeting chimera molecule includes: by taking atoms included in the designated proteolysis targeting chimera molecule as atomic nodes and constructing an adjacency matrix corresponding to the atoms to represent edges between the atomic nodes, obtaining the three-dimensional molecular graph data of the designated proteolysis targeting chimera molecule, wherein the adjacency matrix indicates bonding information between the atoms connected with each other.

In some embodiments, the atomic node of each of the atoms included in the three-dimensional molecular graph data corresponds to node information, and the node information includes at least one of atomic type information, atomic three-dimensional coordinate information, or atomic nuclear charge number.

In some embodiments, for each of the atoms included in the three-dimensional molecular graph data, the atomic type information indicates that which atom type the atom belongs to, and is encoded by a one-hot encoding manner; and the atomic three-dimensional coordinate information indicates three-dimensional coordinate information of the atom in a preset three-dimensional coordinate system determined by projecting the atom to the preset three-dimensional coordinate system, wherein the three-dimensional coordinate system includes a three-dimensional Cartesian coordinate system constructed by taking a molecular centroid of the designated proteolysis targeting chimera molecule as a coordinate origin.

In some embodiments, inputting the three-dimensional molecular graph data of the designated proteolysis targeting chimera molecule into the prediction model to be trained, so that the prediction model predicts the molecular property information of the designated proteolysis targeting chimera molecule includes: by inputting the three-dimensional molecular graph data of the designated proteolysis targeting chimera molecule into an embedding layer of the prediction model, obtaining an embedding vector corresponding to the three-dimensional molecular graph data through the embedding layer; determining a target invariant feature and a target equivariant feature of the designated proteolysis targeting chimera molecule by inputting the embedding vector into an information updating layer of the prediction model; and by inputting a final invariant feature and a final equivariant feature into an output layer of the prediction model, outputting the predicted molecular property information of the designated proteolysis targeting chimera molecule through the output layer.

In some embodiments, by inputting the three-dimensional molecular graph data of the designated proteolysis targeting chimera molecule into the embedding layer of the prediction model, obtaining the embedding vector corresponding to the three-dimensional molecular graph data through the embedding layer includes: for each of atoms in the designated proteolysis targeting chimera molecule, determining atomic feature information of the atom based on the three-dimensional molecular graph data, wherein the atomic feature information includes at least one of atomic type information of the atom, distance information between the atom and other atoms, or adjacent atomic information of the atom; and by inputting the atomic feature information of each of the atoms into the embedding layer of the prediction model, obtaining the embedding vector corresponding to the three-dimensional molecular graph data through the embedding layer.

In some embodiments, the embedding vector includes an initial invariant feature, and determining the target invariant feature and the target equivariant feature of the designated proteolysis targeting chimera molecule by inputting the embedding vector into the information updating layer of the prediction model includes: by the information updating layer, determining an attention weight corresponding to the initial invariant feature, and according to the attention weight, determining an attention feature corresponding to the designated proteolysis targeting chimera molecule; and by the information updating layer, according to the attention feature, determining the target invariant feature and the target equivariant feature of the designated proteolysis targeting chimera molecule.

In some embodiments, by the information updating layer, according to the attention feature, determining the target invariant feature and the target equivariant feature of the designated proteolysis targeting chimera molecule includes: by the information updating layer, obtaining an attention feature corresponding to an initial equivariant feature and an attention feature corresponding to the initial invariant feature, wherein the initial equivariant feature is a zero vector; according to the attention feature corresponding to the initial equivariant feature, updating the attention feature corresponding to the initial invariant feature to obtain an updated initial invariant feature; according to the attention feature corresponding to the initial invariant feature, updating the attention feature corresponding to the initial equivariant feature to obtain an updated initial equivariant feature; by the information updating layer, according to the updated initial invariant feature and the attention feature corresponding to the initial invariant feature, determining the target invariant feature, and according to the updated initial equivariant feature, the updated initial invariant feature and the attention feature corresponding to the initial invariant feature, determining the target equivariant feature.

In some embodiments, in response to determining that there is one information updating layer, taking the target invariant feature and the target equivariant feature are respectively as the final equivariant feature and the final invariant feature; and in response to determining that there are a plurality of information updating layers, taking a target equivariant feature and a target invariant feature determined by a last information updating layer of the prediction model respectively as the final equivariant feature and the final invariant feature.

In the second aspect, the present application provides a method of predicting molecular property information, including: obtaining data of a target proteolysis targeting chimera molecule; according to the data, constructing three-dimensional molecular graph data of the target proteolysis targeting chimera molecule; and inputting the three-dimensional molecular graph data of the target proteolysis targeting chimera molecule into a pre-trained prediction model, so that the prediction model predicts molecular property information of the target proteolysis targeting chimera molecule, wherein the prediction model is trained by the method of the first aspect.

In the third aspect, the present application provides a model training apparatus, including: an obtaining module, configured to obtain data of a designated proteolysis targeting chimera molecule; a constructing module, configured to construct three-dimensional molecular graph data of the designated proteolysis targeting chimera molecule according to the data; a predicting module, configured to input the three-dimensional molecular graph data of the designated proteolysis targeting chimera molecule into a prediction model to be trained, so that the prediction model predicts molecular property information of the designated proteolysis targeting chimera molecule; and a training module, configured to train the prediction model according to a deviation between predicted molecular property information and actual molecular property information of the designated proteolysis targeting chimera molecule.

In the fourth aspect, the present application provides an apparatus of predicting molecular property information, including: an obtaining module, configured to obtain data of a target proteolysis targeting chimera molecule; a constructing module, configured to construct three-dimensional molecular graph data of the target proteolysis targeting chimera molecule according to the data; and a predicting module, configured to input the three-dimensional molecular graph data of the target proteolysis targeting chimera molecule into a pre-trained prediction model, so that the prediction model predicts molecular property information of the target proteolysis targeting chimera molecule, wherein the prediction model is trained by the model training method of the first aspect.

In the fifth aspect, the present application provides a computer-readable storage medium storing a computer program, wherein the computer program is executed by a processor to implement the model training method of the first aspect.

In the sixth aspect, the present application provides a computer-readable storage medium storing a computer program, wherein the computer program is executed by a processor to implement the method of predicting molecular property information of the second aspect.

**In** the seventh aspect, the present application provides an electronic device, including: a memory, a processor, and a computer program stored in the memory and executable on the processor, wherein the processor implements the model training method of the first aspect when executing the computer program.

In the eighth aspect, the present application provides an electronic device, including: a memory, a processor and a computer program stored in the memory and executable on the processor, wherein the processor implements the method of predicting molecular property information of the second aspect when executing the computer program.

At least one of the above technical schemes used in the present application can achieve the following beneficial effects.

As can be seen from the above method, in the present application, three-dimensional molecular graph data of a designated proteolysis targeting chimera can be constructed based on obtained data of the designated proteolysis targeting chimera molecule, where the three-dimensional molecular graph data can fully characterize various features of the molecular structure of the designated proteolysis targeting chimera molecule. And after the three-dimensional molecular graph data is input into a prediction model, the prediction model may, according to the three-dimensional molecular graph data, predict molecular property information of the designated proteolysis targeting chimera molecule. Then the prediction model is trained according to a deviation between predicted molecular property information and actual molecular property information of the designated proteolysis targeting chimera molecule, so that the molecular property information can be obtained quickly and accurately by the prediction model in the subsequent process of predicting the molecular property information, thereby improving the efficiency and accuracy of determining the molecular property information.

## BRIEF DESCRIPTION OF DRAWINGS

The drawings described herein are used to provide further understanding of the present application and constitute a part of the present application. The illustrative embodiments and descriptions thereof of the present application are used to explain the present application and do not constitute improper limitations on the present application. In the drawings:

FIG. 1 illustrates a schematic flowchart of a model training method according to an embodiment of the present application;
FIG. 2 illustrates a schematic flowchart of a molecular property information prediction method according to an embodiment of the present application;
FIG. 3 illustrates a schematic structural diagram of a molecular property prediction system according to an embodiment of the present application;
FIG. 4 illustrates a schematic diagram of a model training apparatus according to an embodiment of the present application;
FIG. 5 illustrates a schematic diagram of a molecular property information prediction apparatus according to an embodiment of the present application;
FIG. 6 illustrates a schematic structural diagram of an electronic device according to an embodiment of the present application.

## DETAILED DESCRIPTION

To make the objectives, technical schemes and advantages of the present application clearer, the technical schemes of the present application will be described clearly and completely with specific embodiments and corresponding drawings of the present application. Apparently, the described embodiments are merely a part rather than all of the embodiments of the present application. Based on the embodiments of the present application, all other embodiments obtained by a person of ordinary skill in the art without creative efforts shall belong to the protection scope of the present application.

In the following, the technical schemes provided by the embodiments of the present application will be described in detail with the drawings.

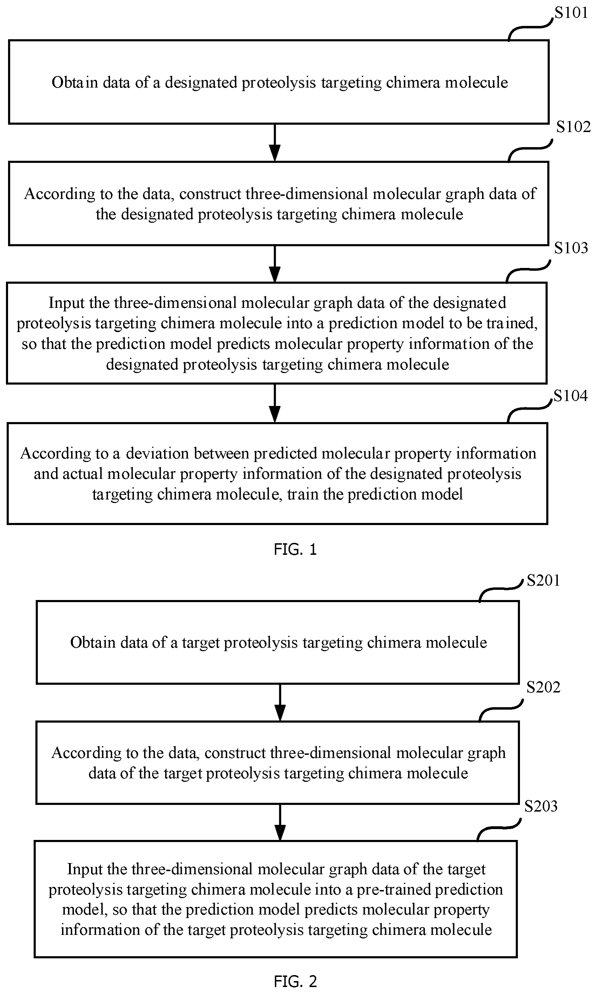

FIG. 1 illustrates a schematic flowchart of a model training method according to an embodiment of the present application. The method includes the following steps.

At S101, data of a designated proteolysis targeting chimera molecule is obtained.

An execution subject of the model training method provided by the present application can be a terminal device such as a desktop computer, a notebook computer, etc., or a server. For the convenience of explanation, in the present application, the terminal device is taken as the execution subject to explain the provided model training method.

In the present application, the terminal device can obtain structural original data of various proteolysis targeting chimera molecules to construct a data set for training the model, where these data can be collected from an external network or obtained in the form of file entry. For example, the original data may include the SMILES (Simplified Molecular-Input Line-Entry System) data set of protein-like degradation targeting chimera molecules and real protein degradation targeting chimera molecules published on the network, and the SMILES data set is a one-dimensional structural representation describing the compound.

After obtaining the data set, the terminal device needs to search for data suitable for training the prediction model from the data set, that is to say, not all the data of various proteolysis targeting chimera molecules included in the data set are suitable for training as samples, some data may not have good labeled molecular fragments, and some data may belong to "dirty data".

Therefore, the terminal device needs to search for proteolysis targeting chimera molecules suitable as training samples from the data set, that is, to determine designated proteolysis targeting chimera molecules. The specific implementation way can be to create an extensible three-dimensional molecular graph structure data generator to clean, reconstruct and optimize the data of protein-like degradation targeting chimera molecules, so as to search out data of a designated proteolysis targeting chimera molecule as a training sample, so that the three-dimensional molecular graph data of the designated proteolysis targeting chimera molecule can be determined in the subsequent process. After cleaning, reconstructing and optimizing the data of protein-like degradation targeting chimera compound, the obtained designated proteolysis targeting chimera has logP value and PK value related to the following invariant feature and equivariant feature, where logP value represents an oil-water partition coefficient of the compound and PK value represents a pharmacokinetic quality.

At S102, according to the data, three-dimensional molecular graph data of the designated proteolysis targeting chimera is constructed.

In the present application, the terminal device can further determine the three-dimensional molecular graph data of the designated proteolysis targeting chimera after determining the data of the designated proteolysis targeting chimera molecule from the above data set. The three-dimensional molecular graph data mentioned here is used to represent structural features of the designated proteolysis targeting chimera molecule, such as a type of each atom, a position of each atom (such as Cartesian coordinates), the nuclear charge number of each atom and the connection relationship between each atom.

For example, each atom included in the designated proteolysis targeting chimera molecule can be taken as an atomic node, and an adjacency matrix corresponding to each atom is constructed to represent edges between the atomic nodes, thus forming a part of the three-dimensional molecular graph data of the designated proteolysis targeting chimera molecule.

The adjacency matrix indicates bonding information between the atoms connected with each other. For example, for two connected atoms, when the two atoms are connected with each other by a single bond, the bonding information may be represented by "0", when the two atoms are connected with each other by a double bond, the bonding information may be represented by "1", when the two atoms are connected with each other by a triple bond, the bonding information may be represented by "2", and when the two atoms are connected with each other by a quadruple bond, the bonding information may be represented by "3".

In some embodiments, for the above three-dimensional molecular graph data, the atomic node of each atom corresponds to node information, and for any atom, the node information corresponding to the atomic node of the atom can be used to represent some inherent characteristics of the atom. For example, the node information corresponding to the atomic node of the atom can include at least one of atomic type information, atomic three-dimensional coordinate information or atomic nuclear charge number.

The atomic type information indicates that which atom type the atom belongs to, such as C, O, S, N, F, Cl, etc. There are many specific manners to determine the atomic type information. For example, the atomic type information can be determined in the form of one-hot encoding. For another example, the atomic type information can be determined by predetermined feature vectors corresponding to element symbols.

For the three-dimensional coordinate information of each atom, each atom can be projected to a preset three-dimensional coordinate system to determine the three-dimensional coordinate information of each atom in the three-dimensional coordinate system. In some examples, the preset three-dimensional coordinate system can be a three-dimensional Cartesian coordinate system constructed with a molecular centroid of the designated proteolysis targeting chimera molecule as a coordinate origin. In other examples, the preset three-dimensional coordinate system can be a three-dimensional coordinate system constructed by taking the first molecule of the designated proteolysis targeting chimera molecule as a coordinate origin. The present application does not limit this.

As can be seen from the above, in the present application, the three-dimensional molecular graph data of the designated proteolysis targeting chimera molecule can be determined by considering the structural characteristics of the designated proteolysis targeting chimera molecule from multiple angles, thus ensuring the accuracy and reasonableness of the prediction results of the prediction model in the subsequent process.

At S103, the three-dimensional molecular graph data of the designated proteolysis targeting chimera molecule is input into a prediction model to be trained, so that the prediction model predicts molecular property information of the designated proteolysis targeting chimera molecule.

In the present application, the prediction model includes an embedding layer, and an embedding vector is obtained based on the input three-dimensional molecular graph data by the embedding layer. And in the subsequent process, the molecular property information can be predicted based on the embedding vector.

In addition, in the present application, the prediction model can further include an information updating layer, whose main function is to obtain an invariant feature and an equivariant feature of the designated proteolysis targeting chimera molecule through multiple data updating operations after the embedding vector is input into the information updating layer, and then input the invariant feature and the equivariant feature into an output layer of the prediction model, so as to output the predicted molecular property information of the designated proteolysis targeting chimera molecule by the output layer.

For each atom in the designated proteolysis targeting chimera molecule, the terminal device can first determine atomic feature information of the atom according to the three-dimensional molecular graph data. The atomic feature information mentioned here indicates the characteristics of the atom, which can include atomic type information of the atom (that is, information mentioned above used to indicate that which atom type the atom belongs to), distance information between the atom and other atoms, and adjacent atomic information of the atom.

In the present application, the distance information between two atoms can be determined in many ways. For example, after determining the three-dimensional coordinate information of each atom, a distance of three-dimensional coordinates between two atoms can be determined, and then the determined distance can be directly used as the distance information between the two atoms. For another example, the distance information between two atoms can be determined by Radial Basis Function (RBF), which can refer to the following formula (1):

$$e^{RBF}(d_{ij}) = \sqrt{\frac{2}{d_c}} \frac{\sin\left(\frac{k\pi}{d_c} d_{ij}\right)}{d_{ij}} \qquad (1)$$

where $d_{ij}$ indicates the distance between atom $i$ and atom $j$, $d_c$ indicates a distance value to a cutoff point, and the specific value can be determined according to the demand; when $d_{ij}$ is equal to $d_c$, the sin function is equal to 0, and $e^{RBF}(d_{ij})$ indicates the distance information between atom $i$ and atom $j$ determined by Radial Basis Function.

The adjacent atomic information mentioned above can be determined by the following embedding formula (2):

$$e_{ij} = embed_1\big(\emptyset_1(A_{ij}) \odot L_1[e^{RBF}(d_{ij})]\big) \qquad (2)$$

where $embed_1$ indicates a Cauchy graph embedding function, $\emptyset_1$ indicates a linear mapping function, $A_{ij}$ indicates a molecular adjacency matrix including bonding information, $L_1$ indicates a linear transformation function, and $e_{ij}$ indicates adjacent atomic information of atom $i$ and atom $j$.

After determining the atomic feature information of each atom, the atomic feature information of each atom can be input into the embedding layer of the prediction model, so as to obtain the embedding vector corresponding to the three-dimensional molecular graph data by the embedding layer. For example, the embedding vector may include the initial invariant feature as shown in the following formula (3-1).

As mentioned above, in the present application, the information updating layer is mainly configured to obtain the invariant feature and the equivariant feature of the proteolysis targeting chimera molecule finally through multiple data updating operations performed by the information updating layer after the embedding vector is input into the information updating layer. In the present application, the information updating layer mainly uses the attention mechanism and message passing mechanism to constantly update the invariant feature and the equivariant feature, and after completing the feature updating, predicts the molecular property information based on the updated feature.

In some embodiments, after the embedding vector is input into the information updating layer of the prediction model, by the information updating layer, an attention weight corresponding to the initial invariant feature can be determined, and according to the attention weight, an attention feature corresponding to the initial invariant feature of the designated proteolysis targeting chimera molecule can be determined. Similarly, after the embedding vector is input into the information updating layer of the prediction model, by the information updating layer, an attention weight corresponding to the initial equivariant feature can be determined, and according to the attention weight, an attention feature corresponding to the initial equivariant feature of the designated proteolysis targeting chimera molecule can be determined. This process can be regarded as all completed in the information updating layer.

The initial invariant feature of the designated proteolysis targeting chimera molecule can be determined by the following formula (3-1):

$$S_i^{(0)} = embed_2\big(\emptyset_2(c_i,\ x_i),\ e_{ij}\big) \qquad (3\text{-}1)$$

where, $c_i$ indicates the nuclear charge number of atom $i$, $x_i$ indicates the atomic type information of atom $i$, $embed_2$ indicates the Laplace characteristic embedding function, $\emptyset_2$ indicates the linear mapping function, and $S_i^{(0)}$ indicates the initial invariant feature of atom $i$.

The initial equivariant feature can be determined by initializing the numerical value. For details, please refer to the following formula (3-2):

$$\vec{V}_i^{(0)} = \vec{0} \qquad (3\text{-}2)$$

As can be seen from the above formula, the initial equivariant feature can be initialized to 0, that is zero vector.

After the initial equivariant feature and the initial invariant feature are determined, these two features can be updated respectively to obtain a target equivariant feature and a target invariant feature.

The prediction model can first obtain query (Q) and key (K) embedding through the attention mechanism in the information updating layer, and query (Q) and key (K) can be determined by the following formulas (4-1) and (4-2):

$$Q = W^Q S + b_Q \qquad (4\text{-}1)$$

$$K = W^K S + b_k \qquad (4\text{-}2)$$

where $W^Q$ indicates a weight coefficient of the attention weight Q corresponding to the invariant feature, $b_Q$ indicates a bias coefficient of the attention weight Q corresponding to the invariant feature, $W^K$ indicates a weight coefficient of the attention weight $K$ corresponding to the invariant feature, $b_k$ indicates a bias coefficient of the attention weight $K$ corresponding to the invariant feature, and S indicates a combination of invariant features. Initially, the invariant feature can be invariant feature $S_i^{(0)}$.

Then, through the attention mechanism in the information updating layer, according to the determined attention weight, the attention feature corresponding to the designated proteolysis targeting chimera molecule can be further determined, as shown in the following formulas (5-1) and (5-2):

$$A_{output} = W_a \sum_i^s Q_i \odot K_i \odot e_i = [y_1,\ y_2,\ y_3\,] \qquad (5\text{-}1)$$

$$\tilde{y}_1 = \emptyset_3\big(\Omega(y_1)\big) \qquad (5\text{-}2)$$

where $W_\alpha$ indicates a weight matrix, $Q_i$ indicates an attention weight Q corresponding to the determined invariant feature of atom $i$, $K_i$ indicates an attention weight $K$ corresponding to the determined invariant feature of atom $i$, $e_i$ indicates adjacent atomic information of atom $i$, $e_i$ is a vector composed by $e_{ij}$ in the above formula (2), and each element is $e_{ij}$ of the j-th atom and the i-th atom. And $y_1$, $y_2$, and $y_3$ have no special practical significance, which can be regarded as splitting a matrix outputted by $A_{output}$ into three matrices of $y_1$, $y_2$, and $y_3$. This is expressed in this way, which is mainly used to determine the invariant features and equivariant features of atoms in the designated proteolysis targeting chimera molecule in the subsequent process. $\Omega$ and $\emptyset_3$ indicate the linear transformation function, through which the $y_1$ is processed, mainly in order to transform $y_1$ in the obtained attention features to a single feature vector.

In the present application, the initial equivariant feature can be updated using the mechanism of VN-MLP equivariant multilayer perceptron. For details, please refer to the following formulas (6-1) and (6-2):

$$Q_V = W \cdot \vec{V} + b_1 \qquad (6\text{-}1)$$

$$K_V = U \cdot \vec{V} + b_2 \qquad (6\text{-}2)$$

where W indicates a weight coefficient of the attention weight $Q_v$ corresponding to the equivariant feature, $b_1$ indicates a bias coefficient of the attention weight $Q_v$ corresponding to the equivariant feature, $U$ indicates a weight coefficient of the attention weight $K_v$ corresponding to the equivariant feature, and $b_2$ indicates a bias coefficient of the attention weight $K_v$ corresponding to the equivariant feature. Initially, $\vec{V}$ can include the equivariant feature $\vec{V}_i^{(0)}$.

Then, the attention feature for the initial equivariant feature can be determined based on the attention weight corresponding to the determined equivariant feature. For details, please refer to the following formula (7):

$$\vec{V} = \begin{cases} Q_V, & \langle Q_V, \ K_V \rangle \geq 0 \\ Q_V - \langle Q_V, \ \frac{K_V}{\|K_V\|} \rangle \cdot \frac{K_V}{\|K_V\|}, & \langle Q_V, \ K_V \rangle < 0 \end{cases} \qquad (7)$$

After the attention feature for the initial invariant feature (also referred to as invariant feature) and the attention feature for the initial equivariant feature (also referred to as equivariant feature) are obtained through the above manner, the invariant feature and equivariant feature can be continuously updated by the information updating layer.

In some embodiments, by the information updating layer, the invariant feature can be updated based on the equivariant feature to obtain the updated invariant feature. Through the above manner, the attention feature $\vec{V}_i$ corresponding to the initial equivariant feature can be determined first, and then the invariant feature $Si$ can be updated based on the attention feature $\vec{V}_i$ corresponding to the initial equivariant feature. For example, the following formulas (8-1) and (8-2) can be used:

$$S'_i = \varphi_1\left(S_i, \ \left\|VN - MLP_1\left(\vec{V}_i\right)\right\|\right) \qquad (8\text{-}1)$$

$$S''_i = \varphi_2\left(S_i, \ \left\|VN - MLP_2(\vec{V}_i)\right\|\right) \qquad (8\text{-}2)$$

where $S'_i$ and $S''_i$ can be regarded as invariant features obtained after two different updates to the invariant feature $S_i$ by using different equivariant functions, and $VN\text{-}MLP_1$ and $VN\text{-}MLP_2$ represent equivariant multilayer perceptron functions. The $\varphi_1$ and $\varphi_2$ in the above formulas represent two different equivariant functions.

For the updating of the equivariant feature, the equivariant feature can be updated according to the invariant feature by the information updating layer, so as to obtain the updated equivariant feature. This process can be as follows: firstly, the attention feature corresponding to the initial equivariant feature is determined according to the initial equivariant feature, and then, the attention feature corresponding to the initial equivariant feature is updated according to the invariant feature $Si$. For details, please refer to the following formula (8-3):

$$\vec{V}_i' = diag\{\varphi_3(S_i)\} \cdot VN - MLP_3(\vec{V}_i) \qquad (8\text{-}3)$$

where $\varphi_3$ indicates an equivariant function, $diag$ indicates a function that transforms a matrix into a diagonal matrix, indicates an updated equivariant feature, which is obtained by updating the attention feature corresponding to the initial equivariant feature in conjunction with the invariant feature $S_i$. And $VN\text{-}MLP_3$ indicates an equivariant multilayer perceptron function.

After obtaining the updated invariant feature, by the information updating layer, the target invariant feature can be determined based on the updated invariant feature $S'_i$ and the determined attention feature $\tilde{y}_1$ corresponding to the initial invariant feature. For details, please refer to the following formula (9-1):

$$m_{ij}^S = ker_1\left(\left\|\vec{r}_{ij}\right\| \odot \tilde{y}_1\right) \odot S'_i \qquad (9\text{-}1)$$

where $ker_1$ indicates a Kelvin function, indicates a distance between atom $i$ and atom $j$, and $m_{ij}^S$ indicates a determined target invariant feature.

For the equivariant feature, the target equivariant feature can be determined according to the updated equivariant feature , the updated invariant feature $S''_i$ and the determined attention features $y_2$ and $y_3$. For details, please refer to the following formula (9-2):

$$m_{ij}^{\vec{V}} = ker_2\left(\left\|\vec{r}_{ij}\right\| \odot y_2\right) \odot \vec{V}_i' + \left\{ker_3\left(\left\|\vec{r}_{ij}\right\| \odot y_3\right) \odot S''_i\right\} \qquad (9\text{-}2)$$

where $ker_2$ and $ker_3$ indicate Kelvin functions, indicates a distance between atom $i$ *and* atom $j$, and $m_{ij}^{\vec{V}}$ indicates the determined target equivariant feature.

It should be noted that the target equivariant feature and the target invariant feature obtained here are the equivariant feature and the invariant feature output by this information updating layer. In some embodiments, in response to determining that there is one information updating layer, the target equivariant feature and the target invariant feature can be directly output to the output layer of the model as the final equivariant feature and the final invariant feature. In some embodiments, in response to determining that there are a plurality of information updating layers, the target equivariant feature and the target invariant feature can further be output to another information updating layer, so as to further update and optimize these features.

In the present application, the prediction model can further include multiple information updating layers. The multiple information updating layers can continuously update and optimize the invariant feature and the equivariant feature through feature transfer among them, so as to finally input the updated and optimized invariant feature and equivariant feature into the output layer of the prediction model.

Therefore, after the above target invariant feature and target equivariant feature are determined, they can be combined into the feature combination of the following formula (10-1):

$$m_i = \left(m_{ij}^S, m_{ij}^{\vec{V}}\right) \qquad (10\text{-}1)$$

Then, the feature combination and the original input are input into the next information updating layer to obtain the output result of the information updating layer, and then the output result is transferred to the next information updating layer, and so on. For details, please refer to the following formula (10-2):

$$\tilde{X} = X_i + f_a(m_i) \qquad (10\text{-}2)$$

In this formula, $m_i$ indicates the feature combination consisting of the target invariant feature of atom $i$ and the target equivariant feature of atom $i$, $X_i$ indicates the atomic feature information of atom $i$, $f_a$ indicates that $m_i$ is processed through the attention mechanism, and $\tilde{X}$ indicates the output result of the information updating layer. It should be pointed out that when determining the target invariant feature of atom $i$, the atomic feature information of atom $j$ connected with atom $i$ is actually considered, and some atom $i$ may not be connected with only one atom $j$. Therefore, when determining the target invariant feature of atom $i$, it is actually possible to summarize $m_{ij}^S$ between atom $i$ *and* each connected atom $j$. The target equivariant feature of atom $i$ is also processed in this way, so as to obtain the feature combination $m_i$ of atom $i$.

The target equivariant feature and the target invariant feature output by the last information updating layer of the prediction model are output to the output layer of the model as the final equivariant feature and the final invariant feature. The output layer predicts the final molecular drug-forming properties by combining the final invariant feature and the final equivariant feature, so as to obtain the molecular property information of the designated proteolysis targeting chimera molecule.

It can be seen from the above that the determined invariant feature of the designated proteolysis targeting chimera molecule actually characterizes the molecular characteristics of the designated proteolysis targeting chimera molecule that will not change with the molecular structure (because the invariant feature of each atom is actually determined by information such as atomic charge number, which is usually fixed under the given molecular structure); and the equivariant feature of the designated proteolysis targeting chimera molecule can characterize the characteristics of some more noteworthy atoms in the molecular structure of the designated proteolysis targeting chimera molecule (because the equivariant feature of each atom is actually determined by the three-dimensional coordinate information of each atom, to simplify the calculation, the initial equivariant feature is set to 0, and the equivariant feature is determined by the attention mechanism).

Therefore, it can be understood that the subsequent output layer actually predicts the molecular property information of the designated proteolysis targeting chimera molecule according to the final equivariant feature and the final invariant feature of the designated proteolysis targeting chimera molecule on the basis of the characteristics of some more noteworthy atoms in the molecular structure of the designated proteolysis targeting chimera molecule and some fixed characteristics of the molecular structure of the designated proteolysis targeting chimera molecule.

At S104, the prediction model is trained according to a deviation between predicted molecular property information and actual molecular property information of the designated proteolysis targeting chimera molecule.

After predicting the above molecular property information, the deviation between the predicted molecular property information and the actual molecular property information of the designated proteolysis targeting chimera molecule can further be determined, and then the prediction model is trained with the optimization goal of minimizing this deviation.

In the present application, the molecular property information may include at least one of: lipophilicity of the designated proteolysis targeting chimera molecule, a pH of the designated proteolysis targeting chimera molecule, a molecular weight of the designated proteolysis targeting chimera molecule, a hydrogen bond donor and a hydrogen bond acceptor of the designated proteolysis targeting chimera molecule, a solubility of the designated proteolysis targeting chimera molecule, or a permeability of the designated proteolysis targeting chimera molecule.

It can be seen from the above methods that in the model training method provided by the present application, the structural features of the molecular structure of the designated proteolysis targeting chimera molecule are considered from multiple angles in the process of determining the three-dimensional molecular graph data of the designated proteolysis targeting chimera molecule. Moreover, because the molecular property information of the designated proteolysis targeting chimera molecule is predicted and determined according to the final invariant feature and the final equivariant feature of the designated proteolysis targeting chimera molecule, the molecular structure characteristics of the designated proteolysis targeting chimera molecule can be fully characterized in this way, thus ensuring that the prediction model can accurately and reasonably predict the molecular property information in the future.

After training the prediction model, the molecular property information of the designated molecule can be predicted by the trained prediction model in practical application. The specific process is shown in FIG. 2.

FIG. 2 illustrates a schematic flowchart of a molecular property information prediction method according to an embodiment of the present application.

At S201, data of a target proteolysis targeting chimera molecule is obtained.

At S202, according to the data, three-dimensional molecular graph data of the target proteolysis targeting chimera molecule is constructed.

In the present application, the terminal device can receive a molecular property prediction instruction of the target proteolysis targeting chimera molecule input by the user, so as to obtain the data of the target proteolysis targeting chimera molecule based on the molecular property prediction instruction, and construct the three-dimensional molecular graph data of the target proteolysis targeting chimera molecule. The molecular property prediction instruction of the target proteolysis targeting chimera molecule may include two fragment molecules provided by the user. The determination of three-dimensional molecular graph data here is basically consistent with the determination process of the three-dimensional molecular graph data in the above model training method, so it will not be described in detail here. The terminal devices mentioned here can refer to desktop computers, notebook computers and other devices.

At S203, the three-dimensional molecular graph data of the target proteolysis targeting chimera molecule is input into a pre-trained prediction model, so that the prediction model predicts molecular property information of the target proteolysis targeting chimera molecule, where the prediction model is trained by the above model training method.

The terminal device can input the three-dimensional molecular graph data of the target proteolysis targeting chimera molecule into the prediction model deployed in the terminal device. The prediction model may output the predicted molecular property information of the target proteolysis targeting chimera molecule.

After the molecular property information is obtained by the prediction model, the molecular property information can be displayed to users, or information can be recommended to users according to the molecular property information, such as recommending pharmaceutical compounds that can be better chimeric with the target proteolysis targeting chimera molecule. Of course, in practical application, the three-dimensional molecular graph data of the target proteolysis targeting chimera molecule can further be stored correspondingly with the predicted molecular property information.

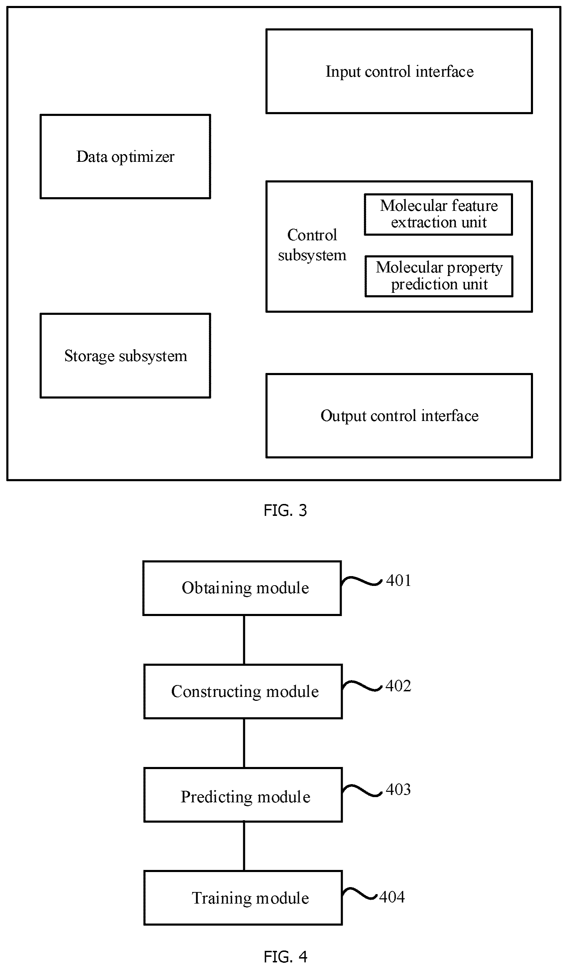

The present application further provides a system for predicting molecular property, as shown in FIG. 3.

FIG. 3 illustrates a schematic structural diagram of a molecular property prediction system according to an embodiment of the present application.

As can be seen from FIG. 3, the system mainly includes the following parts: a storage subsystem, configured to store the above data set and the molecular property information predicted by the prediction model in practical application and the information of their pharmaceutical and chemical properties; a control subsystem, configured to predict the molecular property information of the target proteolysis targeting chimera molecule according to the three-dimensional molecular graph data of the target proteolysis targeting chimera molecule input into the subsystem. The control subsystem includes two units: a molecular feature extraction unit and a molecular property prediction unit, which are respectively configured to determine the atomic feature information of each atom from the three-dimensional molecular graph data and predict the molecular property information of the target proteolysis targeting chimera molecule.

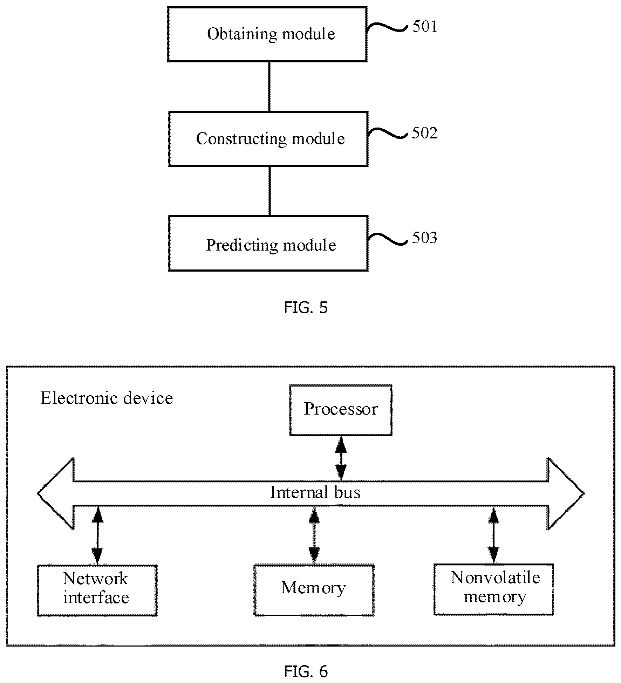

The above are one or more methods implemented in the present application. Based on the same idea, the present application further provides corresponding model training apparatuses and molecular structure information recommendation apparatuses, as shown in FIG. 4 and FIG. 5.

FIG. 4 illustrates a schematic diagram of a model training apparatus according to an embodiment of the present application. The apparatus includes: an obtaining module 401, configured to obtain data of a designated proteolysis targeting chimera molecule; a constructing module 402, configured to construct three-dimensional molecular graph data of the designated proteolysis targeting chimera molecule according to the data; a predicting module 403, configured to input the three-dimensional molecular graph data of the designated proteolysis targeting chimera molecule into a prediction model to be trained, so that the prediction model predicts molecular property information of the designated proteolysis targeting chimera molecule; and a training module 404, configured to train the prediction model according to a deviation between predicted molecular property information and actual molecular property information of the designated proteolysis targeting chimera molecule.

In some embodiments, the molecular property information includes at least one of: lipophilicity of the designated proteolysis targeting chimera molecule, a pH of the designated proteolysis targeting chimera molecule, a molecular weight of the designated proteolysis targeting chimera molecule, a hydrogen bond donor and a hydrogen bond acceptor of the designated proteolysis targeting chimera molecule, a solubility of the designated proteolysis targeting chimera molecule, or a permeability of the designated proteolysis targeting chimera molecule.

In some embodiments, the constructing module 402 is configured to: by taking atoms included in the designated proteolysis targeting chimera molecule as atomic nodes and constructing an adjacency matrix corresponding to the atoms to represent edges between the atomic nodes, obtain the three-dimensional molecular graph data of the designated proteolysis targeting chimera molecule, where the adjacency matrix indicates bonding information between the atoms connected with each other.

In some embodiments, the atomic node of each of the atoms included in the three-dimensional molecular graph data corresponds to node information, and the node information includes at least one of atomic type information, atomic three-dimensional coordinate information, or atomic nuclear charge number.

In some embodiments, for each of the atoms included in the three-dimensional molecular graph data, the atomic type information indicates that which atom type the atom belongs to, and is encoded by a one-hot encoding manner; and the atomic three-dimensional coordinate information indicates three-dimensional coordinate information of the atom in a preset three-dimensional coordinate system determined by projecting the atom to the preset three-dimensional coordinate system, where the three-dimensional coordinate system includes a three-dimensional Cartesian coordinate system constructed by taking a molecular centroid of the designated proteolysis targeting chimera molecule as a coordinate origin.

In some embodiments, the predicting module 403 is configured to: by inputting the three-dimensional molecular graph data of the designated proteolysis targeting chimera molecule into an embedding layer of the prediction model, obtain an embedding vector corresponding to the three-dimensional molecular graph data through the embedding layer; determine a target invariant feature and a target equivariant feature of the designated proteolysis targeting chimera molecule by inputting the embedding vector into an information updating layer of the prediction model; and by inputting a final invariant feature and a final equivariant feature into an output layer of the prediction model, output the predicted molecular property information of the designated proteolysis targeting chimera molecule through the output layer.

In some embodiments, the predicting module 403 is configured to: for each of atoms in the designated proteolysis targeting chimera molecule, determine atomic feature information of the atom based on the three-dimensional molecular graph data, where the atomic feature information includes at least one of atomic type information of the atom, distance information between the atom and other atoms, or adjacent atomic information of the atom; and by inputting the atomic feature information of each of the atoms into the embedding layer of the prediction model, obtain the embedding vector corresponding to the three-dimensional molecular graph data through the embedding layer.

In some embodiments, the embedding vector includes an initial invariant feature, and the predicting module 403 is configured to: by the information updating layer, determine an attention weight corresponding to the initial invariant feature, and according to the attention weight, determine an attention feature corresponding to the designated proteolysis targeting chimera molecule; and by the information updating layer, according to the attention feature, determine the target invariant feature and the target equivariant feature of the designated proteolysis targeting chimera molecule.

In some embodiments, the predicting module 403 is configured to: by the information updating layer, obtain an attention feature corresponding to an initial equivariant feature and an attention feature corresponding to the initial invariant feature, where the initial equivariant feature is a zero vector; according to the attention feature corresponding to the initial equivariant feature, update the attention feature corresponding to the initial invariant feature to obtain an updated initial invariant feature; according to the attention feature corresponding to the initial invariant feature, update the attention feature corresponding to the initial equivariant feature to obtain an updated initial equivariant feature; by the information updating layer, according to the updated initial invariant feature and the attention feature corresponding to the initial invariant feature, determine the target invariant feature, and according to the updated initial equivariant feature, the updated initial invariant feature and the attention feature corresponding to the initial invariant feature, determine the target equivariant feature.

In some embodiments, the predicting module 403 is configured to: in response to determining that there is one information updating layer, take the target invariant feature and the target equivariant feature are respectively as the final equivariant feature and the final invariant feature; and in response to determining that there are a plurality of information updating layers, take a target equivariant feature and a target invariant feature determined by a last information updating layer of the prediction model respectively as the final equivariant feature and the final invariant feature.

FIG. 5 illustrates a schematic diagram of a molecular property information prediction apparatus according to an embodiment of the present application. The apparatus includes: an obtaining module 501, configured to obtain data of a target proteolysis targeting chimera molecule; a constructing module 502, configured to construct three-dimensional molecular graph data of the target proteolysis targeting chimera molecule according to the data; and a predicting module 503, configured to input the three-dimensional molecular graph data of the target proteolysis targeting chimera molecule into a pre-trained prediction model, so that the prediction model predicts molecular property information of the target proteolysis targeting chimera molecule, where the prediction model is trained by the above model training method.

The present application further provides a computer-readable storage medium storing a computer program, where the computer program can be configured to execute the model training method provided in FIG. 1 or the molecular property information prediction method provided in FIG. 2. The computer-readable storage medium may be a nonvolatile storage medium.

The present application further provides a schematic structural diagram of an electronic device shown in FIG. 6 corresponding to FIG. 1 or FIG. 2. As shown in FIG. 6, the electronic device includes a processor, a memory and a nonvolatile memory, and may further include hardware required by other services, such as a network interface and an internal bus. The processor reads the corresponding computer program from the nonvolatile memory into the memory and then runs it to implement the model training method in FIG. 1 or the molecular property information prediction method in FIG. 2.

Of course, besides the software implementation, the present application does not exclude other implementations, such as logic devices or the combination of software and hardware, etc., that is to say, the execution subject of the following processing flow is not limited to logic units, but also can be hardware or logic devices.

In the 1990s, the improvement of a technology can be clearly distinguished as hardware improvement (for example, the improvement of the circuit structure of diodes, transistors, switches, etc.) or software improvement (the improvement of the method flow). However, with the development of technology, the improvement of many current method flows can be regarded as the direct improvement of hardware circuit structure. Designers almost always obtain the corresponding hardware circuit structure by programming the improved method flow into the hardware circuit. Therefore, it cannot be said that the improvement of a method flow cannot be realized by hardware entity modules. For example, Programmable Logic Device (PLD) (such as Field Programmable Gate Array, FPGA) is such an integrated circuit, and its logic function is determined by the user programming the device. Designers program themselves to "integrate" a digital system on a piece of PLD, without the need to ask chip manufacturers to design and produce special integrated circuit chips. Moreover, nowadays, instead of manufacturing integrated circuit chips by hand, this programming is mostly implemented by using "logic compiler" software, which is similar to the software compiler used in program development and writing. The original code before compilation has to be written in a specific programming language, which is called Hardware Description Language (HDL). There is not only one kind of HDL, but many kinds, such as ABEL (Advanced Boolean Expression Language), AHDL (Altera Hardware Description Language), Confluence, CUPL (Cornell University Programming Language), HDCal, JHDL (Java Hardware Description Language), Lava, Lola, MyHDL, PALASM, RHDL (Ruby Hardware Description Language), etc. At present, VHDL (Very-High-Speed Integrated Circuit Hardware Description Language) and Verilog are most commonly used. It should also be clear to those skilled in the art that the hardware circuit implementing the logical method flow can be easily obtained only by logically programming simply the method flow with the above-mentioned hardware description languages and programming it into the integrated circuit.

The controller can be implemented in any suitable way, for example, the controller can take the form of a microprocessor or a processor and a computer-readable medium storing computer-readable program code (such as software or firmware) executable by the (microprocessor) or processor, logic gates, switches, Application Specific Integrated Circuit (ASIC), programmable logic controller and embedded microcontroller. Examples of the controller include but are not limited to the following microcontrollers: ARC 625D, Atmel AT91SAM, Microchip PIC18F26K20 and Silicone Labs C8051F320. The memory controller may further be implemented as a part of the control logic of the memory. Those skilled in the art also know that in addition to implementing the controller in the form of pure computer-readable program codes, the controller can implement the same function in the form of logic gates, switches, application specific integrated circuits, programmable logic controllers, embedded microcontrollers, etc. by logic programming of the method steps. Therefore, this controller can be regarded as a hardware component, and apparatuses for realizing various functions included in this controller can also be regarded as structures in the hardware component. Or even, the apparatuses for realizing various functions can be regarded as both a software module for implementing the method and a structure within a hardware component.

The systems, apparatuses, modules or units set forth in the above embodiments can be implemented by computer chips or entities, or by products with certain functions. A typical implementation device is a computer. Specifically, the computer can be, for example, a personal computer, a laptop computer, a cellular phone, a camera phone, a smart phone, a personal digital assistant, a media player, a navigation device, an email device, a game console, a tablet computer, a wearable device or a combination of any of these devices.

For the convenience of description, the above apparatus is divided into various units based on functions and then described separately. Of course, the functions of units can be realized in one or more pieces of software and/or hardware when the present application is implemented.

It should be understood by those skilled in the art that embodiments of the present application can be provided as a method, a system, or a computer program product. Therefore, the present application can take the form of an entirely hardware embodiment, an entirely software embodiment, or an embodiment combining software and hardware aspects. Moreover, the present application can take the form of a computer program product implemented on one or more computer-usable storage media (including but not limited to disk storage, CD-ROM, optical storage, etc.) containing computer-usable program codes.

The present application is described with reference to flowcharts and/or block diagrams of methods, devices (systems), and computer program products according to embodiments of the present application. It should be understood that each flow and/or block in the flowchart and/or block diagram, and combinations of the flows and/or blocks in the flowchart and/or block diagram can be implemented by computer program instructions. These computer program instructions may be provided to a processor of a general-purpose computer, special-purpose computer, embedded processor or other programmable data processing devices to produce a machine, such that the instructions which are executed by the processor of the computer or other programmable data processing devices produce apparatus for implementing the functions specified in flow or flows in the flowchart and/or block or blocks in the block diagram.

These computer program instructions may also be stored in a computer-readable memory that can direct a computer or other programmable data processing devices to function in a particular manner, such that the instructions stored in the computer-readable memory produce a manufacture article including instruction means that implement the functions specified in flow or flows in the flowchart and/or block or blocks in the block diagram.

These computer program instructions may also be loaded onto a computer or other programmable data processing devices, such that a series of operational steps are performed on the computer or other programmable devices to produce a computer-implemented process, such that the instructions executed on the computer or other programmable devices provide steps for implementing the functions specified in flow or flows in the flowchart and/or block or blocks in the block diagram.

In a typical configuration, a computing device includes one or more processors (CPU), input/output interfaces, a network interface, and a memory.

The memory may include non-permanent memory, random access memory (RAM) and/or nonvolatile memory in computer-readable media, such as read-only memory (ROM) or flash RAM. The memory is an example of a computer-readable medium.

The computer-readable media include permanent and non-permanent, removable and non-removable media. Information storage can be realized by any method or technology. Information can be computer-readable instructions, data structures, and modules of programs or other data. Examples of storage media of a computer include, but not limited to, phase change memory (PRAM), static random access memory (SRAM), dynamic random access memory (DRAM), other types of random access memory (RAM), read-only memory (ROM), electrically erasable programmable read-only memory (EEPROM), flash memory or other memory technologies, read-only optical disk read-only memory (CD-ROM), digital versatile disc (DVD) or other optical storage, magnetic tape, magnetic tape disk storage or other magnetic storage device or any other non-transmission medium, and these storage media can be used to store information that can be accessed by the computing device. According to the definition in the present application, the computer-readable media do not include transitory computer-readable media, such as modulated data signals and carrier waves.

It should also be noted that the terms "including", "containing" or any other variation thereof are intended to cover non-exclusive inclusion, so that a process, method, commodity or device including a series of elements includes not only those elements, but also other elements not explicitly listed, or elements inherent to such process, method, commodity or device. Without more restrictions, an element defined by the phrase "including a..." does not exclude the existence of other identical elements in the process, method, commodity or device including the element.

Those skilled in the art should appreciate that embodiments of the present application may be provided as methods, systems or computer program products. Therefore, the present application can take the form of an entirely hardware embodiment, an entirely software embodiment or an embodiment combining software and hardware aspects. Moreover, the present application can take the form of a computer program product implemented on one or more computer-usable storage media (including but not limited to disk storage, CD-ROM, optical storage, etc.) containing computer-usable program codes.

The present application may be described in the general context of computer-executable instructions, such as program modules, being executed by a computer. Generally, the program modules include routines, programs, objects, components, data structures, etc. that perform particular tasks or implement particular abstract data types. The present application may also be practiced in distributed computing environments where tasks are performed by remote processing devices that are connected through a communication network. In a distributed computing environment, the program modules may be located in local and remote computer storage media including storage devices.

The embodiments in the present application are described in a progressive way, and the same and similar parts between the embodiments can be referred to each other, and each embodiment focuses on the differences from other embodiments. Especially, for the system embodiment, because it is basically similar to the method embodiment, the description is relatively simple, and the relevant points can be found in the partial description of the method embodiment.

The above are only examples of the present application, and are not used to limit the present application. For those skilled in the art, the present application may be subject to various modifications and variations. Any modification, equivalent substitution, improvement, etc. made within the spirit and principle of the present application shall be included in the scope of the claims of the present application.

**Claims**

1. A model training method, comprising:

   obtaining data of a designated proteolysis targeting chimera molecule;
   according to the data, constructing three-dimensional molecular graph data of the designated proteolysis targeting chimera molecule;
   inputting the three-dimensional molecular graph data of the designated proteolysis targeting chimera molecule into a prediction model to be trained, so that the prediction model predicts molecular property information of the designated proteolysis targeting chimera molecule; and
   according to a deviation between predicted molecular property information and actual molecular property information of the designated proteolysis targeting chimera molecule, training the prediction model.

2. The method of claim 1, wherein the molecular property information comprises at least one of:

   lipophilicity of the designated proteolysis targeting chimera molecule,
   a pH of the designated proteolysis targeting chimera molecule,
   a molecular weight of the designated proteolysis targeting chimera molecule,
   a hydrogen bond donor and a hydrogen bond acceptor of the designated proteolysis targeting chimera molecule,
   a solubility of the designated proteolysis targeting chimera molecule, or
   a permeability of the designated proteolysis targeting chimera molecule.

3. The method of claim 1, wherein according to the data, constructing the three-dimensional molecular graph data of the designated proteolysis targeting chimera molecule comprises:
   by taking atoms comprised in the designated proteolysis targeting chimera molecule as atomic nodes and constructing an adjacency matrix corresponding to the atoms representing edges between the atomic nodes, obtaining the three-dimensional molecular graph data of the designated proteolysis targeting chimera molecule, wherein the adjacency matrix indicates bonding information between the atoms connected with each other.

4. The method of claim 3, wherein the atomic node of each of the atoms comprised in the three-dimensional molecular graph data corresponds to node information, and
   the node information comprises at least one of atomic type information, atomic three-dimensional coordinate information, or atomic nuclear charge number.

5. The method of claim 4, wherein for each of the atoms comprised in the three-dimensional molecular graph data,

   the atomic type information indicates that which atom type the atom belongs to, and is encoded by a one-hot encoding manner; and
   the atomic three-dimensional coordinate information indicates three-dimensional coordinate information of the atom in a preset three-dimensional coordinate system determined by projecting the atom to the preset three-dimensional coordinate system, wherein the three-dimensional coordinate system comprises a three-dimensional Cartesian coordinate system constructed by taking a molecular centroid of the designated proteolysis targeting chimera molecule as a coordinate origin.

6. The method of claim 1, wherein inputting the three-dimensional molecular graph data of the designated proteolysis targeting chimera molecule into the prediction model to be trained, so that the prediction model predicts the molecular property information of the designated proteolysis targeting chimera molecule comprises:

by inputting the three-dimensional molecular graph data of the designated proteolysis targeting chimera molecule into an embedding layer of the prediction model, obtaining an embedding vector corresponding to the three-dimensional molecular graph data through the embedding layer;
determining a target invariant feature and a target equivariant feature of the designated proteolysis targeting chimera molecule by inputting the embedding vector into an information updating layer of the prediction model; and
by inputting a final invariant feature and a final equivariant feature into an output layer of the prediction model, outputting the predicted molecular property information of the designated proteolysis targeting chimera molecule through the output layer.

7. The method of claim 6, wherein by inputting the three-dimensional molecular graph data of the designated proteolysis targeting chimera molecule into the embedding layer of the prediction model, obtaining the embedding vector corresponding to the three-dimensional molecular graph data through the embedding layer comprises:

for each of atoms in the designated proteolysis targeting chimera molecule, determining atomic feature information of the atom based on the three-dimensional molecular graph data, wherein the atomic feature information comprises at least one of atomic type information of the atom, distance information between the atom and other atoms, or adjacent atomic information of the atom; and
by inputting the atomic feature information of each of the atoms into the embedding layer of the prediction model, obtaining the embedding vector corresponding to the three-dimensional molecular graph data through the embedding layer.

8. The method of claim 6, wherein the embedding vector comprises an initial invariant feature, and determining the target invariant feature and the target equivariant feature of the designated proteolysis targeting chimera molecule by inputting the embedding vector into the information updating layer of the prediction model comprises:

by the information updating layer, determining an attention weight corresponding to the initial invariant feature, and according to the attention weight, determining an attention feature corresponding to the designated proteolysis targeting chimera molecule; and
by the information updating layer, according to the attention feature, determining the target invariant feature and the target equivariant feature of the designated proteolysis targeting chimera molecule.

9. The method of claim 8, wherein by the information updating layer, according to the attention feature, determining the target invariant feature and the target equivariant feature of the designated proteolysis targeting chimera molecule comprises:

by the information updating layer, obtaining an attention feature corresponding to an initial equivariant feature and an attention feature corresponding to the initial invariant feature, wherein the initial equivariant feature is a zero vector;
according to the attention feature corresponding to the initial equivariant feature, updating the attention feature corresponding to the initial invariant feature to obtain an updated initial invariant feature;
according to the attention feature corresponding to the initial invariant feature, updating the attention feature corresponding to the initial equivariant feature to obtain an updated initial equivariant feature;
by the information updating layer, according to the updated initial invariant feature and the attention feature corresponding to the initial invariant feature, determining the target invariant feature, and according to the updated initial equivariant feature, the updated initial invariant feature and the attention feature corresponding to the initial invariant feature, determining the target equivariant feature.

10. The method of claim 6, wherein,

in response to determining that there is one information updating layer, taking the target invariant feature and the target equivariant feature are respectively as the final equivariant feature and the final invariant feature; and
in response to determining that there are a plurality of information updating layers, taking a target equivariant feature and a target invariant feature determined by a last information updating layer of the prediction model respectively as the final equivariant feature and the final invariant feature.

11. A method of predicting molecular property information, comprising:

   obtaining data of a target proteolysis targeting chimera molecule;
   according to the data, constructing three-dimensional molecular graph data of the target proteolysis targeting chimera molecule; and
   inputting the three-dimensional molecular graph data of the target proteolysis targeting chimera molecule into a pre-trained prediction model, so that the prediction model predicts molecular property information of the target proteolysis targeting chimera molecule, wherein the prediction model is trained by the method of any one of claims 1 to 10.

12. A model training apparatus, comprising:

   an obtaining module, configured to obtain data of a designated proteolysis targeting chimera molecule;
   a constructing module, configured to construct three-dimensional molecular graph data of the designated proteolysis targeting chimera molecule according to the data;
   a predicting module, configured to input the three-dimensional molecular graph data of the designated proteolysis targeting chimera molecule into a prediction model to be trained, so that the prediction model predicts molecular property information of the designated proteolysis targeting chimera molecule; and
   a training module, configured to train the prediction model according to a deviation between predicted molecular property information and actual molecular property information of the designated proteolysis targeting chimera molecule.

13. An apparatus of predicting molecular property information, comprising:

   an obtaining module, configured to obtain data of a target proteolysis targeting chimera molecule;
   a constructing module, configured to construct three-dimensional molecular graph data of the target proteolysis targeting chimera molecule according to the data; and
   a predicting module, configured to input the three-dimensional molecular graph data of the target proteolysis targeting chimera molecule into a pre-trained prediction model, so that the prediction model predicts molecular property information of the target proteolysis targeting chimera molecule, wherein the prediction model is trained by the method of any one of claims 1 to 10.

14. A computer-readable storage medium storing a computer program, wherein the computer program is executed by a processor to implement the method of any one of claims 1 to 10.

15. A computer-readable storage medium storing a computer program, wherein the computer program is executed by a processor to implement the method of claim 11.

16. An electronic device, comprising: a memory, a processor, and a computer program stored in the memory and executable on the processor, wherein the processor implements the method of any one of claims 1 to 10 when executing the computer program.

17. An electronic device, comprising: a memory, a processor and a computer program stored in the memory and executable on the processor, wherein the processor implements the method of claim 11 when executing the computer program.

S101

Obtain data of a designated proteolysis targeting chimera molecule

S102

According to the data, construct three-dimensional molecular graph data of the designated proteolysis targeting chimera molecule

S103

Input the three-dimensional molecular graph data of the designated proteolysis targeting chimera molecule into a prediction model to be trained, so that the prediction model predicts molecular property information of the designated proteolysis targeting chimera molecule

S104

According to a deviation between predicted molecular property information and actual molecular property information of the designated proteolysis targeting chimera molecule, train the prediction model

FIG. 1

S201

Obtain data of a target proteolysis targeting chimera molecule

S202

According to the data, construct three-dimensional molecular graph data of the target proteolysis targeting chimera molecule

S203

Input the three-dimensional molecular graph data of the target proteolysis targeting chimera molecule into a pre-trained prediction model, so that the prediction model predicts molecular property information of the target proteolysis targeting chimera molecule

FIG. 2

FIG. 3

FIG. 4

FIG. 5

FIG. 6

## INTERNATIONAL SEARCH REPORT

| International application No. |
| --- |
| **PCT/CN2024/075814** |

| A. | CLASSIFICATION OF SUBJECT MATTER |
| --- | --- |

G16B15/30(2019.01)i;  G16B15/20(2019.01)i;  G16B40/00(2019.01)i

According to International Patent Classification (IPC) or to both national classification and IPC

| B. | FIELDS SEARCHED |
| --- | --- |

Minimum documentation searched (classification system followed by classification symbols)

Documentation searched other than minimum documentation to the extent that such documents are included in the fields searched

IPC: G16B

Electronic data base consulted during the international search (name of data base and, where practicable, search terms used)

CNTXT, DWPI, ENTXT, ENTXTC, VEN, WPABS, WPABSC, PUBMED, ISI WEB OF SCIENCE: 之江实验室, 蛋白降解靶向嵌合体, 模型, 嵌入层, 三维坐标, 训练, 分子性质, protein degradation target chimera molecule, model training, three-dimensional molecular diagram data, embedding Layer, predict

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116524998 A (ZHEJIANG LAB) 01 August 2023 (2023-08-01) abstract, and claims 1-20 | 1-17 |
| PX | CN 116597892 A (ZHEJIANG LAB) 15 August 2023 (2023-08-15) abstract, and claims 1-18 | 1-17 |
| X | US 2022415452 A1 (TENCENT TECHNOLOGY (SHENZHEN) CO., LTD.) 29 December 2022 (2022-12-29) description, paragraphs 8-16 | 1-17 |
| X | CN 112289371 A (BEIJING STONEWISE TECHNOLOGY CO., LTD.) 29 January 2021 (2021-01-29) description, paragraphs 4-21 | 1-17 |
| X | CN 113241128 A (TIANJIN UNIVERSITY) 10 August 2021 (2021-08-10) description, paragraphs 27-39 | 1-17 |
| X | CN 115512785 A (OCEAN UNIVERSITY OF CHINA) 23 December 2022 (2022-12-23) description, paragraphs 9-65 | 1-17 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **06 May 2024** | **15 May 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**EP 4 708 301 A1**

**INTERNATIONAL SEARCH REPORT**

| International application No. |
|---|
| **PCT/CN2024/075814** |

**C.    DOCUMENTS CONSIDERED TO BE RELEVANT**

| Category* | Citation of document, with indication, where appropriate, of the relevant passages | Relevant to claim No. |
|---|---|---|
| A | CN 114171125 A (SUN YAT-SEN UNIVERSITY) 11 March 2022 (2022-03-11)<br>    entire document | 1-17 |
| A | CN 113160894 A (PING AN TECHNOLOGY (SHENZHEN) CO., LTD.) 23 July 2021<br>(2021-07-23)<br>    entire document | 1-17 |
| A | US 2020392178 A1 (INTERNATIONSAL BUSINESS MACHINES CORPORATION) 17<br>December 2020 (2020-12-17)<br>    entire document | 1-17 |
| A | 谢良旭等 (XIE, Liangxu et al.). "神经网络的深度与宽度对药物分子pKa 预测性能影响的研究 (Research on Impact of Depth and Width of Neural Network on pKa Prediction Performance of Drug Molecules"<br>江苏理工学院学报 (Journal of Jiangsu University of Technology).<br>Vol. 27, No. 2, 30 April 2021 (2021-04-30),<br>    pages 1-8 | 1-17 |
| A | ZHAN, Xinke et al. "Predicting Protein–Protein Interactions Based on Ensemble Learning-Based Model from Protein Sequence"<br>*Biology*, Vol. 11, No. 995, 30 June 2022 (2022-06-30),<br>    pages 1-14 | 1-17 |

Form PCT/ISA/210 (second sheet) (July 2022)

## INTERNATIONAL SEARCH REPORT
### Information on patent family members

International application No.

**PCT/CN2024/075814**

| Patent document cited in search report | | | Publication date (day/month/year) | Patent family member(s) | | | Publication date (day/month/year) |
|---|---|---|---|---|---|---|---|
| CN | 116524998 | A | 01 August 2023 | None | | | |
| CN | 116597892 | A | 15 August 2023 | None | | | |
| US | 2022415452 | A1 | 29 December 2022 | WO | 2022022173 | A1 | 03 February 2022 |
| CN | 112289371 | A | 29 January 2021 | None | | | |
| CN | 113241128 | A | 10 August 2021 | None | | | |
| CN | 115512785 | A | 23 December 2022 | None | | | |
| CN | 114171125 | A | 11 March 2022 | None | | | |
| CN | 113160894 | A | 23 July 2021 | None | | | |
| US | 2020392178 | A1 | 17 December 2020 | None | | | |

Form PCT/ISA/210 (patent family annex) (July 2022)